# DEMANDE DE BREVET EUROPEEN

(11) **EP 1 430 876 A1**
(43) Date de publication de la demande: **23.06.2004**
(21) Numéro de dépôt: 03293258.4
(22) Date de dépôt: 19.12.2003
(51) Int. Cl.: A61K 7/13

(54) **Composition pour coloration de fibres kératiniques contenant un colorant direct et un polymère particulier, procédé de préparation de cette composition et procédé de coloration avec ladite composition.**

(30) Priorité: 19.12.2002 FR 0216205
(71) Demandeur: L'OREAL, 75008 Paris (FR)
(72) Inventeur: Guerin, Frédéric, 75009 Paris (FR); Samain, Henri, 91570 Bièvres (FR)
(74) Mandataire: Dossmann, Gérard

(57) **Abrégé**

L'objet de l'invention concerne une composition pour la teinture des fibres kératiniques, en particulier des fibres kératiniques humaines telles que les cheveux humains contenant dans un milieu approprié au moins un colorant direct particulier et au moins un polymère hydrosoluble sous forme dispersée ou sous forme solubilisée de PM moyen en poids supérieur à 10⁶, ledit polymère étant obtenu et introduit dans la composition sous forme d'une dispersion issue de la polymérisation dans une solution aqueuse saline d'au moins un monomère hydrosoluble comportant au moins une double liaison en présence :
- d'au moins un agent dispersant constitué par un (poly)électrolyte soluble
- et d'au moins un agent empêchant l'augmentation de la viscosité,
ainsi qu'un procédé de préparation des compositions colorantes, des procédés de coloration des fibres kératiniques incorporant ces composés et des dispositifs de teinture ou "kits" à plusieurs compartiments.

## Description

L'invention est relative à l'utilisation pour la teinture des fibres kératiniques, en particulier des fibres kératiniques humaines telles que les cheveux humains d'une composition contenant au moins un colorant direct particulier et au moins un polymère particulier sous forme dispersée ou sous forme solubilisée, un procédé de préparation de ces compositions ainsi qu'un procédé de coloration direct utilisant ces compositions.

Aujourd'hui les produits de coloration capillaire permettent de changer la couleur de cheveux pour quelques heures, quelques jours, quelques semaines, quelques mois avec un retour à l'état initial ou une persistance du résultat, tout en respectant l'intégrité de la fibre.

Plusieurs familles d'agents de coloration ont été développées pour atteindre ces différents objectifs en termes de couleurs et de durabilité. La coloration capillaire semi-permanente ou fugace est réalisée classiquement en utilisant des colorants directs. Elle consiste à colorer les cheveux en faisant pénétrer une molécule colorée par diffusion à l'intérieur du cheveu. Selon leur ténacité ou leur rapidité à s'estomper, les colorants directs utilisés dans les formules de coloration, permettent d'obtenir des montées de couleurs chromatiques cependant très sélectives et de plus peu tenaces aux shampooings.

Plusieurs procédés d'obtention de polymères hydrosolubles sous forme de dispersion existent dans l'art antérieur. Le brevet FR 2 815 635 porte sur un procédé de préparation d'une dispersion aqueuse d'un polymère permettant de limiter les variations de viscosité du mélange réactionnel tout au long de la polymérisation.

Le brevet FR 2 812 295 porte sur un procédé de préparation d'un polymère cationique de haut poids moléculaire à base de sel de dialkyle et de diallyle ammonium sous forme de perles par la technique de polymérisation en suspension inverse.

La demande WO 02/40622 porte sur l'utilisation d'une solution aqueuse d'un copolymère cationique à base d'un monomère non-ionique et d'un monomère cationique dans une composition d'un détergent.

La demande FR 2 816 833 décrit l'utilisation en cosmétique d'un polymère hydrosoluble sous forme d'une dispersion susceptible d'être obtenu par polymérisation d'au moins un monomère hydrosoluble comportant au moins une double liaison dans une solution aqueuse saline.

La demanderesse a trouvé de manière surprenante et avantageuse que l'introduction de polymères particuliers qui sont sous forme dispersée ou sous forme solubilisée dans les compositions de coloration de fibres kératiniques contenant un colorant direct particulier permettait d'obtenir une baisse de la sélectivité, des montées et des ténacités de couleurs supérieures à celles obtenues en l'absence de ce polymère sous forme dispersée ou solubilisée.

De plus, l'introduction de dispersions de polymères particuliers dans les compositions de coloration de fibres kératiniques contenant un colorant direct permettait de produire des effets cosmétiques supérieurs aux résultats obtenus avec des compositions similaires ne contenant pas ces polymères. Elles permettent ainsi d'obtenir un meilleur effet conditionneur de la fibre

Ces compositions de coloration de fibres kératiniques sont de plus capables de produire d'autres effets potentiellement intéressants tels que l'épaississement de la composition à la dilution.

Par ailleurs la demanderesse a découvert que sous forme dispersée dans la composition tinctoriale, le polymère particulier pouvait protéger les colorants directs des dégradations liées à des agents extérieurs. Cette protection est encore plus importante si les colorants directs sont introduits lors de la synthèse des polymères sous forme dispersée. Il convient par ailleurs de noter que si la dilution de la dispersion initiale dans la composition finale est suffisante, elle conduira à la solubilisation des particules polymériques hydrodispersées. L'invention concerne également un procédé de préparation des compositions colorantes, des procédés de coloration des fibres kératiniques incorporant ces composés, ainsi que des dispositifs de teinture ou "kits" à plusieurs compartiments.

D'autres objets, caractéristiques, aspects et avantages de l'invention apparaîtront encore plus clairement à la lecture de la description et des divers exemples qui suivent.

La présente invention a pour objet une composition pour la teinture des fibres kératiniques, en particulier des fibres kératiniques humaines telles que les cheveux humains contenant dans un milieu approprié :
- au moins un colorant direct particulier,
- et au moins un polymère hydrosoluble sous forme dispersée ou sous forme solubilisée de PM moyen en poids supérieur à 10⁶, ledit polymère étant obtenu et introduit dans la composition sous forme d'une dispersion issue de la polymérisation dans une solution aqueuse saline d'au moins un monomère hydrosoluble comportant au moins une double liaison en présence :

- d'au moins un agent dispersant constitué par un (poly)électrolyte soluble,
- et d'au moins un agent empêchant l'augmentation de la viscosité.

La composition contient au moins un colorant direct choisi parmi les colorants directs nitrés benzèniques, les colorants directs azoïques, les colorants directs quinoniques et en particulier anthraquinoniques et naphtoquinoniques, les colorants directs aziniques, les colorants directs méthiniques et azométhiniques, les colorants directs triarylméthaniques, les colorants directs dérivés des phénols, des aminophénols, des naphtols, les colorants directs dérivés des porphyrines, en particulier les tétraphénylporphyrines, des métalloporphyrines, les colorants directs indoaminiques, les colorants xanthèniques, les colorants directs naturels.

Les colorants directs peuvent être de nature non ionique, cationique, anionique ou amphotère.

Ils peuvent être aussi fluorescents.

Parmi les colorants directs benzèniques, on peut citer notamment les composés suivants:
- 1,4-diamino-2-nitrobenzène
- 1-amino-2 nitro-4-β- hydroxyéthylaminobenzène
- 1-amino-2 nitro-4-bis(β-hydroxyéthyl)-aminobenzène
- 1,4-Bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques, on peut citer les colorants azoïques cationiques décrits dans les demandes de brevets WO 95/15144, WO 95/01772 et EP 714954. Les passages de ces demandes relatives aux colorants directs azoïques font partie intégrante de l'invention.

Parmi ces composés, on peut tout particulièrement citer les colorants suivants :
- chlorure de 1,3-diméthyl-2-[[4-(diméthylamino)phényl]azo]-1H-imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3^{e} édition :
- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

On peut aussi citer le 1-(4'-aminodiphénylazo)-2-méthyl-4bis-(β-hydroxyéthyl)aminobenzène et l'acide 4-hydroxy-3-(2-méthoxyphénylazo)-1-naphialène sulfonique.

Parmi les colorants directs quinoniques, on peut citer les colorants suivants :
- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99
ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques, on peut citer les composés suivants :
- Basic Blue 17
- Basic Red 2.

Parmi les colorants triarylméthaniques, on peut citer les composés suivants :
- Basic Green 1
- Acid blue 9
- Basic Violet 3
- Basic Violet 14
- Basic Blue 7
- Acid Violet 49
- Basic Blue 26
- Acid Blue 7

Parmi les colorants indoaminiques, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Selon l'invention, le ou les colorants directs utilisés représentent de préférence, de 0,001% à 10% en poids environ du poids total de la composition tinctotriale et encore plus préférentiellement de 0,005% à 5% en poids environ.

Il est à noter, que les colorants directs peuvent être introduits lors de la polymérisation et/ou après celle-ci.

La partie de la demande EP 0 943 628 de l'art antérieur concernant l'obtention du polymère sous forme d'une dispersion fait partie intégrante de l'invention.

Par polymère hydrosoluble sous forme dispersée ou sous forme solubilisée, on entend au sens de la présente invention un polymère potentiellement hydrosoluble, c'est-à-dire dont la solubilité potentielle dans l'eau est supérieure à 0,1% en poids à 25°C (évaluation visuelle) et qui peut être utilisé in fine sous forme soluble ou dispersée.

Lesdits polymères utilisés dans le cadre de la présente invention sont avantageusement obtenus à partir de monomères hydrosolubles comportant au moins une double liaison. Ces derniers peuvent être choisis parmi des monomères cationiques, anioniques, non-ioniques et leurs mélanges.

A titre de monomères anioniques, on peut notamment citer l'acide (méth)acrylique, l'acide acrylamido-2-méthylpropanesulfonique, l'acide itaconique et leurs sels d'un métal alcalin, d'alcalinoterreux ou d'ammonium ou ceux issus d'une amine organique telle que l'alcanolamine.

A titre de monomères non-ioniques, on peut notamment citer parmi le (méth)acrylamide, le N-vinylformamide, le N-vinylacétoamide et le (méth)acrylate d'hydroxypropyle.

Les monomères cationiques sont, de préférence, choisis parmi les sels d'ammonium quaternaire dérivés d'une diallylamine, et ceux répondant à la formule suivante (I) : 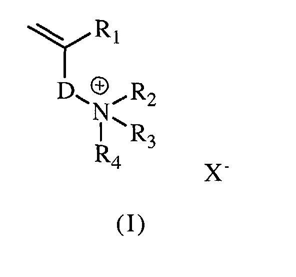 dans laquelle :
R₁ représente un atome d'hydrogène ou un groupement méthyle,
R₂ et R₃, identiques ou différents, représentent un atome d'hydrogène ou un groupe alkyle en C₁-C₄ linéaire ou ramifié,
R₄ représente un atome d'hydrogène, un groupe alkyle en C₁-C₄ linéaire ou ramifié ou un groupe aryle,
D représente le motif suivant, 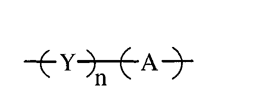
dans lequel :
Y représente une fonction amide, un ester (O-CO ou CO-O), un uréthane ou une urée,
A représente un groupement alkylène en C₁-C₁₀ linéaire ou ramifié, cyclique ou acyclique, pouvant être substitué ou interrompu par un groupement aromatique ou hétéroaromatique divalent. Les groupements alkylènes peuvent être interrompus par un atome d'oxygène, un atome d'azote, un atome de soufre ou un atome de phosphore ; l'alkylène pouvant contenir une fonction cétone, un amide, un ester (O-CO ou CO-O), un uréthane, ou une urée,
n est un nombre entier variant de 0 à 1,
X⁻ représente un contre ion anionique, comme par exemple un chlorure ou un sulfate.

A titre d'exemples de monomères cationiques hydrosolubles, on peut notamment citer le chlorhydrate ou le sulfate dérivé du (méth)acrylate de diméthylaminoéthyle, le chlorure de (méth)acryloyloxyéthyltriméthylammonium, le chlorure de (méth)acryloyloxyéthyldiméthylbenzylammonium, le chlorhydrate ou le sulfate dérivé du N-[diméthylaminopropyl](méth)acrylamide, le chlorure de (méth)acrylamidopropyltriméthylammonium, le chlorure de (méth)acrylamidopropyldiméthylbenzylammonium, le chlorhydrate ou le sulfate dérivé du (méth)acrylate de diméthylaminohydroxypropryle, le chlorure de (méth)acryloyloxyhydroxypropyltriméthylammonium, le chlorure de (méth)acryloyloxyhydroxypropyldiméthylbenzylammonium et le chlorure de diméthyldiallylammonium.

De préférence, le polymère selon l'invention est polymérisé à partir d'au moins un monomère cationique tel que défini ci-dessus.

De préférence, les polymères sont polymérisés à partir des monomères comportant au moins une double liaison suivants :
- 0 à 30% en moles d'acide acrylique,
- 0 à 95,5% en moles d'acrylamide et,
- 0,5 à 100% en moles d'au moins un monomère cationique représenté dans la formule (I) telle que définie ci-dessus.

Selon un mode de réalisation particulier, les polymères sont polymérisés à partir d'un monomère cationique et d'acide acrylique, le nombre de mole du monomère cationique étant supérieur au nombre de mole d'acide acrylique.

Comme polymères particulièrement préférés dans l'invention, on peut notamment citer ceux polymérisés à partir de
- 10% de chlorure d'acryloyloxyéthyldiméthylbenzylammonium et de 90% d'acrylamide,
- 30% de chlorure d'acryloyloxytriméthylammonium, 50% de chlorure d'acryloyloxyéthyldiméthylbenzylammonium, et de 20 % d'acrylamide,
- 10% de chlorure d'acryloyloxyéthyltriméthylammonium et de 90% d'acrylamide
- 30% de chlorure de diallyldiméthylammonium et de 70% d'acrylamide
- 30% d'acide acrylique et de 70% d'acrylamide.

Les polymères décrits ci-dessus ont plus particulièrement un poids moléculaire en poids supérieur à 1 000 000 et de préférence entre 1 000 000 et 50 000 000. Le poids moléculaire est déterminé par la méthode RSV (Reduced Specific Viscocity) tel que définie dans "Principles of Polymer Chemistry" Cornell University Press, Ithaca, NY 1953 Chapter VII "Determination of molecular Weight" pp 266-316.

Dans ces compositions, les polymères de l'invention peuvent être présents à une concentration comprise entre 0,05% et 10%, de préférence entre 0,1% et 5%, et encore plus préférentiellement entre 0,2% et 2% en poids par rapport au poids total de la composition.

L'agent dispersant utilisé dans la présente invention, est un (poly)électrolyte, de préférence un (poly)électrolyte cationique obtenu par polymérisation de 50 à 100% en moles d'au moins un monomère cationique choisi parmi les sels, par exemple, les chlorhydrates ou les sulfates, dérivés du (méth)acrylate de diméthylaminoéthyle, du N-[diméthylaminopropyl](méth)acrylamide, ou d'une di(méth)allylamine, le chlorure de (méth)acryloyloxyéthyltriméthylammonium, le chlorure de (méth)acrylamidopropyltriméthylammonium, le chlorure de diméthyldiallylammonium, et leurs mélanges, et de 50 à 0% en moles d'acrylamide. On peut utiliser en outre une polyamine telle qu'une polyalkylèneamine.

Selon l'invention, la solution aqueuse saline qui sert de milieu de dispersion au polymère est une solution aqueuse saline permettant de solubiliser les monomères hydrosolubles comportant des doubles liaisons et l'agent dispersant, mais ne solubilisant pas le polymère obtenu.

Cette solution aqueuse saline comprend au moins un sel, de préférence un sel anionique divalent tel que, par exemple, le sulfate d'ammonium, l'hydrogénosulfate d'ammonium, le sulfate de sodium, l'hydrogénosulfate de sodium, le sulfate de magnésium, l'hydrogéno-sulfate de magnésium, le sulfate d'aluminium, l'hydrogénosulfate d'aluminium. Le sulfate d'ammonium et le sulfate de sodium sont particulièrement préférés.

Ces sels peuvent être utilisés en une concentration allant de 15 % en poids jusqu'à leurs concentrations de saturation de manière à ce qu'ils présentent les propriétés mentionnées ci-dessus. La solution aqueuse peut contenir en outre des sels anioniques monovalents, comme le chlorure de sodium et le chlorure d'ammonium.

L'agent empêchant l'augmentation de la viscosité du milieu réactionnel lors de la polymérisation est choisi parmi :
(A) les acides polycarboxyliques ou leurs sels,
(B) les polyphénols,
(C) les composés cycliques contenant un groupe hydroxy et un groupe carboxy, ou leurs sels,
(D) l'acide gluconique ou ses sels,
(E) les produits réactionnels obtenus par réaction d'une méthoxyhydroquinone et/ou d'un monomère cationique (méth)acrylique avec un composé qui génère des radicaux, sous une atmosphère oxydante,
(F) les produits réactionnels obtenus par réaction d'un polymère cationique (méth)acrylique avec un composé qui génère des radicaux, sous une atmosphère oxydante,
(G) les produits réactionnels obtenus par réaction d'un polymère cationique (méth)acrylique avec un oxydant, et
leurs mélanges.

L'utilisation d'un tel milieu réactionnel permet d'obtenir, lorsque le milieu est agité lors de la polymérisation, une dispersion stable de polymère.

L'addition d'au moins un agent empêchant l'augmentation de la viscosité (A) à (G), tels que décrits ci-dessus permet d'effectuer la polymérisation des monomères hydrosolubles tels que décrits ci-dessus, avec un agitateur à faible vitesse tout en évitant la formation de particules grossières. Il est préférable que les agents empêchant l'augmentation de la viscosité (A) à (G) soient solubles dans l'eau, mieux encore dissous dans la solution aqueuse saline qui sert de milieu de dispersion.

Comme exemples de composé (A), on peut citer l'acide oxalique, l'acide adipique, l'acide tartrique, l'acide malique, l'acide phtalique et leurs sels.

Comme exemples de composé (B), on peut notamment citer le résorcinol et le pyrogallol.

Comme exemples de composé (C), on peut notamment citer l'acide m-hydroxybenzoïque, l'acide p-hydroxybenzoïque, l'acide salicylique, l'acide gallique, l'acide tannique et leurs sels.

Comme exemples de composé (D), on peut notamment citer le gluconate de sodium, le gluconate de potassium, le gluconate d'ammonium, et différents sels aminés de l'acide gluconique.

Comme exemples de composé (E), on peut notamment citer ceux obtenus en laissant réagir un composé qui génère des radicaux, sous un courant de gaz oxygéné, dans une solution contenant la méthoxyhydroquinone et/ou un monomère cationique (méth)acrylique. Le composé qui génère des radicaux peut être un amorceur de polymérisation couramment utilisé dans la polymérisation radicalaire. On peut notamment citer un amorceur de polymérisation azoïque hydrosoluble tel que le chlorhydrate de 2,2'-azobis(2-amidinopropane), vendu par exemple sous la dénomination V-50 par la société Wako Chemical Industries, ou le chlorhydrate de 2,2'-azobis[2-(2-imidazoline-2-yl)propane] vendu par exemple sous la dénomination commerciale VA-044 par la société Wako Chemical Industries, ou un amorceur de polymérisation de type oxydoréducteur hydrosoluble tel que l'association persulfate d'ammonium/hydrogénosulfite de sodium.

On peut obtenir un agent empêchant l'augmentation de la viscosité (F) par réaction d'un amorceur de polymérisation qui est un composé qui génère des radicaux, sous une atmosphère oxydante, par exemple sous un courant de gaz oxygéné, avec un agent dispersant selon l'invention. L'amorceur de polymérisation peut être un amorceur de polymérisation azoïque hydrosoluble tel que cité ci-dessus, ou un amorceur de polymérisation de type oxydoréducteur hydrosoluble tel que l'association persulfate d'ammonium/hydrogénosulfite de sodium.

L'agent empêchant l'augmentation de la viscosité (G) peut être obtenu sous la forme d'un polymère oxydé de faible masse moléculaire, par oxydation d'un agent dispersant selon l'invention obtenu par polymérisation d'un monomère cationique (méth)acrylique, avec du peroxyde d'hydrogène ou un halogène comme oxydant.

Comme monomères cationiques (méth)acryliques utilisés pour la préparation des agents empêchant l'augmentation de la viscosité (E), (F) et (G), on peut citer, par exemple, le chlorhydrate ou le sulfate dérivé du (méth)acrylate de diméthylaminoéthyle, le chlorure de (méth)acryloyloxyéthyltriméthylammonium, le chlorure de (méth)acryloyloxyéthyldiméthylbenzylammonium, le chlorhydrate ou le sulfate dérivé du N[diméthylaminopropyl](méth)acrylamide, le chlorure de (méth)acrylamidopropyltriméthylammonium, le chlorure ou le sulfate dérivé du (méth)acrylate de diméthylaminohydroxypropyle, le chlorure de (méth)acryloyloxyhydroxypropyltriméthylammonium, le chlorure de (méth)acryloyloxyhydroxypropyldiméthylbenzylammonium.

Ces agents empêchant l'augmentation de la viscosité (A) à (G) peuvent être utilisés seuls ou en mélange, en une quantité comprise de préférence entre 10 ppm et 10 000 ppm par rapport au poids de la solution réactionnelle.

Le milieu approprié pour la composition pour la coloration de fibres kératiniques est de préférence un milieu aqueux constitué par de l'eau ou par un mélange d'eau et d'un solvant cosmétiquement acceptable, choisi de préférence parmi les alcools inférieurs en C₁-C₄, l'alcool benzylique, les éthers de polyols, les alcanes en C₅-C₁₀, les cétones en C₃-C₄, les acétates d'alkyles en C₁-C₄, le diméthoxyéthane, et leurs mélanges.

Le pH des compositions de l'invention est compris genéralement entre 3 et 11, de préférence entre 4 et 10.

Les additifs cosmétiques sont choisis parmi des agents tensio-actifs anioniques, cationiques, non-ioniques ou amphotères, des agents épaississants non polymériques comme des acides ou des électrolytes, des agents antioxydants, des agents dispersants, des agents séquestrants, des parfums, des agents de conditionnement, des agents conservateurs, des agents opacifiants, des agents alcalins, des agents acides ainsi que tout autre adjuvant utilisé habituellement en teinture de matières kératiniques.

L'homme du métier veillera à choisir les éventuels additifs et leur quantité de manière à ce qu'ils ne nuisent pas aux propriétés des compositions de la présente invention.

Ces additifs sont présents dans la composition selon l'invention en une quantité allant de 0 à 20% en poids par rapport au poids total de la composition.

Cette composition appliquée sur les cheveux peut se présenter sous des formes galéniques diverses, telles qu'une lotion, une crème, un gel ou sous tout autre forme appropriée pour réaliser une teinture des fibres kératiniques. Elle peut également être conditionnée sous pression en flacon aérosol en présence d'un agent propulseur et former une mousse.

Dans le cas des aérosols, le propulseur utilisé peut être constitué par les gaz liquéfiés ou comprimés usuellement employés pour la préparation de compositions aérosols. On emploiera, de manière préférentielle, l'air, le gaz carbonique, l'azote comprimé ou encore un gaz liquéfiable à température ordinaire tel que le diméthyléther, les hydrocarbures halogénés (fluorés en particulier) ou non, et leurs mélanges.

La présente invention concerne également un procédé de préparation des compositions de l'invention consistant à introduire dans un milieu contenant au moins un colorant direct tel que défini ci-dessus, au moins un polymère hydrosoluble tel que décrit précédemment, sous forme dispersée et de PM moyen supérieur à 10⁶, ladite dispersion étant obtenue par polymérisation dans une solution aqueuse saline d'au moins un monomère hydrosoluble comportant au moins une double liaison en présence :
- d'au moins un agent dispersant constitué par un (poly)électrolyte soluble,
- et d'au moins un agent empêchant l'augmentation de la viscosité.

Une variante de ce procédé consiste à introduire au moins un colorant direct selon l'invention au moment de la préparation de la dispersion de polymère et d'introduire la dispersion contenant le ou les colorants directs dans le milieu final de la composition tinctoriale.

La présente invention concerne également un procédé de coloration direct des matières kératiniques en particulier les cheveux, tel que la composition décrite ci-dessus est appliquée sur les fibres kératiniques. Le ou les colorants directs utilisables selon l'invention et le ou les polymères mis en oeuvre dans l'invention peuvent être appliqués sur les fibres de manière simultanée ou de manière séquentielle. Cela signifie que la composition selon l'invention est formée par deux compositions, l'une A contenant au moins un colorant direct tel que défini ci-dessus et l'autre B contenant au moins un polymère hydrosoluble tel que défini auparavant sous forme dispersée ou solubilisée, la composition A pouvant être appliquée avant la composition B ou inversement, la composition selon l'invention étant ainsi formée directement sur les fibres. Le temps d'application est compris généralement entre 3 et 60 minutes et préférentiellement entre 5 à 40 minutes. La température d'application est comprise de préférence entre la température ambiante et 80°C, préférentiellement entre 25°C et 55°C.

L'invention concerne également un dispositif à 2 compartiments pour la teinture des fibres kératiniques en particulier de fibres kératiniques humaines et plus particulièrement les cheveux, tel qu'un premier compartiment renferme une composition colorante contenant, au moins un colorant direct tel que défini ci-dessus, et qu'un autre compartiment renferme une composition contenant dans un milieu approprié pour la teinture, au moins un polymère hydrosoluble tel que décrit auparavant sous forme dispersée ou solubilisée et de préférence sous forme dispersée, ledit polymère étant obtenu par polymérisation dans une solution aqueuse saline d'au moins un monomère hydrosoluble comportant au moins une double liaison contenant au moins un agent dispersant constitué par un (poly)électrolyte soluble, et au moins un agent empêchant l'augmentation de la viscosité. Ainsi, une fois les deux compositions renfermées dans ce dispositif mélangées, on obtient la composition selon l'invention.

## Revendications

1. Composition pour la teinture des fibres kératiniques, en particulier des fibres kératiniques humaines telles que les cheveux humains contenant dans un milieu approprié:
- au moins un colorant direct choisi parmi les colorants directs nitrés benzèniques, les colorants directs azoïques, les colorants directs quinoniques, les colorants directs aziniques, les colorants directs méthiniques et azométhiniques, les colorants directs triarylméthaniques, les colorants directs dérivés des phénols, des aminophénols, des naphtols, les colorants directs dérivés des porphyrines, les colorants directs indoaminiques, les colorants xanthèniques, les colorants directs naturels,
- et au moins un polymère hydrosoluble sous forme dispersée ou sous forme solubilisée de PM moyen en poids supérieur à 10⁶, ledit polymère étant obtenu et introduit dans la composition sous forme d'une dispersion issue de la polymérisation dans une solution aqueuse saline d'au moins un monomère hydrosoluble comportant au moins une double liaison en présence :
- d'au moins un agent dispersant constitué par un (poly)électrolyte soluble,
- et d'au moins un agent empêchant l'augmentation de la viscosité.

2. Composition selon la revendication 1 **caractérisée par le fait que** le colorant direct est de nature non ionique, cationique, anionique ou amphotère.

3. Composition selon la revendication 1 ou 2, **caractérisée par le fait que** le colorant est fluorescent.

4. Composition selon l'une quelconque des revendications 1 à 3,
**caractérisée par le fait que** le colorant direct est un colorant direct benzènique nitré choisi parmi :
- 1,4-diamino-2-nitrobenzène
- 1-amino-2 nitro-4-β- hydroxyéthylaminobenzène
- 1-amino-2 nitro-4-bis(β-hydroxyéthyl)-aminobenzène
- 1,4-Bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

5. Composition selon l'une quelconque des revendications 1 à 3,
**caractérisée par le fait que** le colorant direct est un colorant direct azoïque choisi parmi :
- chlorure de 1,3-diméthyl-2-[[4-(diméthylamino)phényl]azo]-1H-imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium
- Di sperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.
- 1-(4'-aminodiphénylazo)-2-méthyl-4bis-(β-hydroxyéthyl)amino-benzène
- acide 4-hydroxy-3-(2-méthoxy-phénylazo)-1-naphtalène sulfonique.

6. Composition selon l'une quelconque des revendications 1 à 3,
**caractérisée par le fait que** le colorant direct est un colorant direct quinonique choisi parmi :
- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

7. Composition selon l'une quelconque des revendications 1 à 3,
**caractérisée par le fait que** le colorant direct est un colorant azinique choisi parmi:
- Basic Blue 17
- Basic Red 2.

8. Composition selon l'une quelconque des revendications 1 à 3,
**caractérisée par le fait que** le colorant direct est un colorant triarylméthanique choisi parmi :
- Basic Green 1
- Acid blue 9
- Basic Violet 3
- Basic Violet 14
- Basic Blue 7
- Acid Violet 49
- Basic Blue 26
- Acid Blue 7

9. Composition selon l'une quelconque des revendications 1 à 3,
**caractérisée par le fait que** le colorant direct est un colorant indoaminique choisi parmi :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

10. Composition selon l'une quelconque des revendications 1 à 3, **caractérisée par le fait que** le colorant direct est un colorant direct naturel choisi parmi : la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine.

11. Composition selon l'une quelconque des revendications 1 à 10, **caractérisée en ce que** le ou les colorants directs utilisés représentent de préférence, de 0,001% à 10% en poids environ du poids total de la composition tinctotriale et encore plus préférentiellement de 0,005% à 5% en poids environ.

12. Composition selon l'une quelconque des revendications 1 à 11, **caractérisée en ce que** le ou les monomères hydrosolubles comportant au moins une double liaison sont choisis parmi des monomères cationiques, anioniques, non anioniques et leurs mélanges.

13. Composition selon la revendication 12, **caractérisée en ce que** les monomères anioniques sont choisis parmi l'acide (méth)acrylique, l'acide acrylamido-2-méthylpropanesulfonique, l'acide itaconique et leurs sels d'un métal alcalin, d'alcalinoterreux ou d'ammonium ou ceux issus d'une amine organique telle que l'alcanolamine.

14. Composition selon la revendication 12, **caractérisée en ce que** les monomères non ioniques sont choisis parmi le (méth)acrylamide, le N-vinylformamide, le N-vinylacétoamide et le (méth)acrylate d'hydroxypropyle.

15. Composition selon la revendication 12, **caractérisée en ce que** les monomères cationiques sont choisis parmi les sels d'ammonium quaternaire dérivés d'une diallylamine, et ceux répondant à la formule suivante (I) : 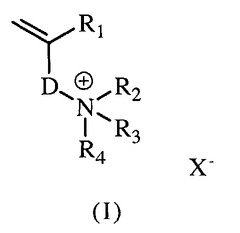 dans laquelle :
R₁ représente un atome d'hydrogène ou un groupement méthyle,
R₂ et R₃, identiques ou différents, représentent un atome d'hydrogène ou un groupe alkyle en C₁-C₄ linéaire ou ramifié,
R₄ représente un atome d'hydrogène, un groupe alkyle en C₁-C₄ linéaire ou ramifié, un groupe aryle,
D représente le motif suivant, 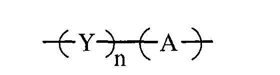
dans lequel :
Y représente une fonction amide, un ester (O-CO ou CO-O), un uréthane ou une urée,
A représente un groupement alkylène en C₁-C₁₀ linéaire ou ramifié, cyclique ou acyclique, pouvant être substitué ou interrompu par un aromatique ou un hétéroaromatique divalent, les groupements alkylènes pouvant être interrompus par un atome d'oxygène, un atome d'azote, un atome de soufre ou un atome de phosphore ; l'alkylène pouvant contenir une cétone, un amide, un ester (O-CO ou CO-O), un uréthane, ou une urée,
n est un nombre entier variant de 0 à 1,
X⁻ représente un contre ion anionique, comme un chlorure ou un sulfate.

16. Composition selon l'une quelconque des revendications 1 à 15, **caractérisée par le fait que** le polymère est polymérisé à partir d'au moins un monomère cationique tel que défini à la revendication 15.

17. Composition selon l'une quelconque des revendications de 1 à 16, **caractérisée en ce que** le polymère est polymérisé à partir des monomères comportant au moins une double liaison suivants :
- 0 à 30% en moles d'acide acrylique,
- 0 à 95,5% en moles d'acrylamide et,
- 0,5 à 100% en moles d'au moins un monomère cationique de formule (I) telle que définie dans la revendication 15.

18. Composition selon la revendication 17, **caractérisée en ce que** le polymère est polymérisé à partir d'un monomère cationique et d'acide acrylique, le nombre de mole du monomère cationique étant supérieur au nombre de mole d'acide acrylique.

19. Composition selon la revendication 17, **caractérisée en ce que** le polymère est polymérisé à partir de 10% de chlorure d'acryloyloxyéthyldiméthylbenzylammonium et de 90% d'acrylamide,

20. Composition selon la revendication 17, **caractérisée en ce que** le polymère est polymérisé à partir de 30% de chlorure d'acryloyloxytriméthylammonium, 50% de chlorure d'acryloyloxyéthyldiméthylbenzylammonium, et de 20 % d'acrylamide,

21. Composition selon la revendication 17, **caractérisée en ce que** le polymère est polymérisé à partir de 10% de chlorure d'acryloyloxyéthyltriméthylammonium et de 90% d'acrylamide,

22. Composition selon la revendication 17, **caractérisée en ce que** le polymère hydrosoluble est polymérisé à partir de 30% de chlorure de diallyldiméthylammonium et de 70% d'acrylamide,

23. Composition selon l'une quelconque des revendications 1 à 14, **caractérisée en ce que** le polymère est polymérisé à partir de 30% d'acide acrylique et de 70% d'acrylamide.

24. Composition selon l'une quelconque des revendications 1 à 23, **caractérisée en ce que** le ou les polymères de l'invention sont présents à une concentration comprise entre 0,05% et 10%, de préférence entre 0,1% et 5%, et encore plus préférentiellement entre 0,2% et 2% en poids par rapport au poids total de la composition.

25. Composition selon l'une quelconque des revendications 1 à 24, **caractérisée en ce que** l'agent dispersant est un (poly)électrolyte cationique obtenu par polymérisation de 50 à 100% en moles d'au moins un monomère cationique choisi parmi les sels dérivés du (méth)acrylate de diméthylaminoéthyle, du N-[diméthylaminopropyl]-(méth)acrylamide, ou d'une di(méth)allylamine, le chlorure de (méth)acryloyloxytriméthylammonium, le chlorure de (méth)acrylamidopropyltriméthylammonium, le chlorure de diméthyldiallylammonium, et leurs mélanges, et de 50 à 0% en moles d'acrylamide.

26. Composition selon l'une quelconque des revendications 1 à 25, **caractérisée en ce que** la solution aqueuse saline comprend au moins un sel anionique divalent.

27. Composition selon la revendication 26, **caractérisée en ce que** le sel anionique divalent est choisi parmi le sulfate d'ammonium, l'hydrogénosulfate d'ammonium, le sulfate de sodium, l'hydrogénosulfate de sodium, le sulfate de magnésium, l'hydrogénosulfate de magnésium, le sulfate d'aluminium, l'hydrogénosulfate d'aluminium.

28. Composition selon l'une quelconque des revendications 1 à 27, **caractérisée en ce que** l'agent empêchant l'augmentation de la viscosité du milieu réactionnel lors de la polymérisation est choisi parmi :
(A) les acides polycarboxyliques ou leurs sels,
(B)les polyphénols,
(C)les composés cycliques contenant un groupe hydroxy et un groupe carboxy, ou leurs sels,
(D)l'acide gluconique ou ses sels,
(E)les produits réactionnels obtenus par réaction d'une méthoxyhydroquinone et/ou d'un monomère cationique (méth)acrylique avec un composé qui génère des radicaux, sous une atmosphère oxydante,
(F)les produits réactionnels obtenus par réaction d'un polymère cationique (méth)acrylique avec un composé qui génère des radicaux, sous une atmosphère oxydante,
(G)les produits réactionnels obtenus par réaction d'un polymère cationique (méth)acrylique avec un oxydant, et
leurs mélanges.

29. Composition selon la revendication 28, **caractérisée en ce que** l'agent empêchant l'augmentation de la viscosité (A) est choisi parmi l'acide oxalique, l'acide adipique, l'acide tartrique, l'acide malique, l'acide phtalique et leurs sels.

30. Composition selon la revendication 28, **caractérisée en ce que** l'agent empêchant l'augmentation de la viscosité (B) est choisi parmi le résorcinol et le pyrogallol.

31. Composition selon la revendication 28, **caractérisée en ce que** l'agent empêchant l'augmentation de la viscosité (C) est choisi parmi l'acide m-hydroxybenzoïque, l'acide p-hydroxybenzoïque, l'acide salicylique, l'acide gallique, l'acide tannique et leurs sels.

32. Composition selon la revendication 28, **caractérisée en ce que** l'agent empêchant l'augmentation de la viscosité (D) est choisi parmi le gluconate de sodium, le gluconate de potassium, le gluconate d'ammonium, et différents sels aminés de l'acide gluconique.

33. Composition selon l'une quelconque des revendications 1 à 32, **caractérisée en ce que** le milieu approprié pour la composition pour la coloration de fibres kératiniques est de préférence un milieu aqueux constitué par de l'eau ou par un mélange d'eau et d'un solvant cosmétiquement acceptable.

34. Composition selon la revendication 33, **caractérisée en ce que** le solvant approprié est choisi parmi les alcools inférieurs en C₁-C₄, l'alcool benzylique, les éthers de polyols, les alcanes en C₅-C₁₀, les cétones en C₃-C₄, les acétates d'alkyles en C₁-C₄, le diméthoxyéthane, et leurs mélanges.

35. Composition selon l'une quelconque des revendications 1 à 34, telle qu'elle comprend des additifs cosmétiques choisis parmi des agents tensio-actifs anioniques, cationiques, non-ioniques ou amphotères, des agents épaississants non polymériques comme des acides ou des électrolytes, des agents antioxydants, des agents dispersants, des agents séquestrants, des parfums, des agents de conditionnement, des agents conservateurs, des agents opacifiants, des agents alcalins, des agents acides.

36. Composition selon l'une quelconque des revendications 1 à 35, **caractérisée en ce que** le polymère hydrosoluble introduit sous forme dispersée est solubilisée.

37. Composition selon l'une quelconque des revendications 1 à 36, **caractérisée en ce qu'**elle se présente sous la forme d'une lotion, d'une crème, d'un gel.

38. Composition selon l'une quelconque des revendications 1 à 37, **caractérisée en ce qu'**elle est conditionnée en aérosol.

39. Procédé de préparation de composition **caractérisé par le fait qu'**il consiste à introduire dans un milieu contenant au moins un colorant direct tel que défini dans les revendications 1 à 11 et au moins un polymère hydrosoluble sous forme dispersée ou sous forme solubilisée de PM moyen supérieur à 10⁶, ledit polymère étant obtenu et introduit dans la composition sous forme d'une dispersion issue de la polymérisation dans une solution aqueuse saline d'au moins un monomère hydrosoluble comportant au moins une double liaison en présence :
- d'au moins un agent dispersant constitué par un (poly)électrolyte soluble,
- et d'au moins un agent empêchant l'augmentation de la viscosité.

40. Procédé de préparation de composition, **caractérisée par le fait qu'**il consiste à introduire dans un milieu cosmétiquement acceptable au moins une dispersion d'un polymère hydrosoluble de PM moyen en poids supérieur à 10⁶, ledit polymère étant obtenu et introduit dans la composition sous forme d'une dispersion issue de la polymérisation dans une solution aqueuse saline d'au moins un monomère hydrosoluble comportant au moins une double liaison en présence :
- d'au moins un agent dispersant constitué par un (poly)électrolyte soluble,
- et d'au moins un agent empêchant l'augmentation de la viscosité,
et contenant au moins un colorant direct tel que défini dans les revendications 1 à 11, et au moins un.

41. Procédé de teinture des fibres kératiniques et en particulier fibres kératiniques humaines, et plus particulièrement les cheveux, **caractérisé par le fait qu'**il consiste à appliquer sur les fibres une composition telle que définie dans les revendications 1 à 38 ; le temps d'application étant compris généralement entre 3 et 60 minutes et préférentiellement entre 5 à 40 minutes ; la température d'application étant comprise de préférence entre la température ambiante et 80°C préférentiellement entre 25°C et 55°C.

42. Procédé selon la revendications 41, **caractérisé par le fait qu'**il consiste à appliquer sur les fibres une composition formée par une composition A contenant au moins un colorant direct tel que défini dans les revendications 1 à 11, et par une composition B contenant au moins un polymère hydrosoluble sous forme dispersée ou solubilisée de PM moyen en poids supérieur à 10⁶; la composition A étant appliquée sur les fibres avant la composition B.

43. Procédé selon la revendication 41, **caractérisé par le fait qu'**il consiste à appliquer sur les fibres une composition formée par une composition A contenant au moins un colorant direct tel que défini dans les revendications 1 à 11, et par une composition B contenant au moins un polymère hydrosoluble sous forme de dispersée ou solubilisée de PM moyen en poids supérieur à 10⁶ ; la composition B étant appliquée sur les fibres avant la composition A.

44. Dispositif à 2 compartiments pour la teinture des fibres kératiniques en particulier de fibres kératiniques humaines et plus particulièrement les cheveux, **caractérisé par le fait qu'**un premier compartiment renferme une composition colorante contenant, dans un milieu approprié pour la teinture, au moins un colorant direct tel que défini dans les revendications 1 à 11, et qu'un autre compartiment renferme une composition contenant au moins un polymère hydrosoluble sous forme de dispersée ou solubilisée de PM moyen en poids supérieur à 10⁶, ledit polymère étant obtenu par polymérisation dans une solution aqueuse saline d'au moins un monomère hydrosoluble comportant au moins une double liaison contenant au moins un agent dispersant constitué par un (poly)électrolyte soluble, et au moins un agent empêchant l'augmentation de la viscosité.
